# FASCICULE DE BREVET EUROPEEN

(11) **EP 0 646 578 B1**
(45) Date de publication et mention de la délivrance du brevet: **31.05.2000**
(21) Numéro de dépôt: 94402183.1
(22) Date de dépôt: 29.09.1994
(51) Int. Cl.: C07D 261/18, A61K 31/42

(54) **Phényl carboxamides isoxazoles et leurs sels, leur procédé de préparation, leur application comme médicaments et les compositions pharmaceutiques les renfermant**
Phenyl-Carboxamide-Isoxazole-Derivate und Salze, Verfahren zu ihrer Herstellung ihre Anwendung als Arzneimittel und pharmazeutische Zusammensetzungen die sie enthalten
Phenyl carboxamide-isoxazole-derivatives and salts, process for their preparation, their use as pharmaceuticals, and pharmaceutical compositions containing them

(30) Priorité: 01.10.1993 GB 9320299
(43) Date de publication de la demande: 05.04.1995
(73) Titulaire: HOECHST MARION ROUSSEL, 92800 Puteaux (FR)
(72) Inventeur: Kuo, Elizabeth Anne, Swindon, Wiltshire SN3 5PH (GB); Westwood, Robert, Kingston Bagpuize, Oxon (GB)
(74) Mandataire: Vieillefosse, Jean-Claude

(56) Documents cités:
- EP-A- 0 326 108
- FR-A- 2 313 052

## Description

L'invention concerne de nouveaux phénylcarboxamide-isoxazoles et leurs sels, leur procédé de préparation, les compositions pharmaceutiques les contenant et leur utilisation comme médicaments.

WO91/17748 décrit certains dérivés d'isoxazole et leur utilisation comme médicaments.

EP-A-326107 divulgue des bêta-cycloalkyl-bêta-oxopropionitriles anti-arthritiques et leur méthode de préparation.

EP-A-326108 et FR-A-2313031 divulguent certains dérivés d'isoxazole substitués par alkyle.

EP-A-440503 décrit des dérivés d'isoxazole substitués par trois radicaux différents et pouvant être utlisés pour le traitement de maladies inflammatoires.

L'invention a pour objet des composés de formule (I) : 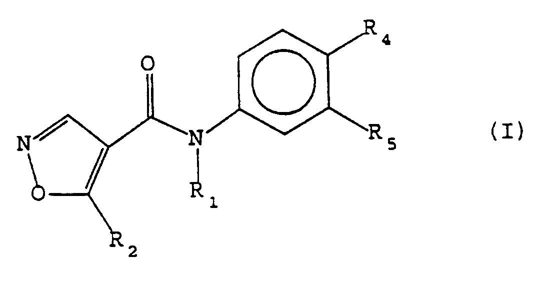 [dans laquelle
R₁ représente un atome d'hydrogène ou un groupement alkyle contenant 1 à 3 atomes de carbone;
R₂ représente un groupement cycloalkyle contenant 3 à 6 atomes de carbone;
R₄ représente un atome d'halogène, un groupement nitrile, un groupement nitro, un groupement -SCH₃, un groupement alkylcarbonyle en C₁₋₆, un groupement cycloalkylcarbonyle en C₃₋₆, un groupement -CX₃, un groupement -WCX₃, un groupement -(CH₂)ₙCX₃, un groupement -(CX₂)ₙCX₃, un groupement -W(CX₂)ₙCX₃ ou un groupement -W(CH₂)ₙCX₃, dans lesquels X représente un atome d'halogène, W représente un atome d'oxygène ou de soufre, et n représente 1, 2 ou 3; et
R₅ représente un groupement méthyle ;
leurs formes tautomères ainsi que leurs sels.

Le terme groupement cycloalkyle contenant 3 à 6 atomes de carbone désigne un groupement cyclopropyle, cyclobutyle, cyclopentyle ou cyclohexyle.

Le terme groupement alkyle contenant 1 à 3 atomes de carbone désigne un groupement méthyle, éthyle, propyle ou isopropyle.

Le terme atome d'halogène désigne un atome de fluor, de chlore, de brome ou d'iode.

Les composés préférés selon l'invention sont ceux dans lesquels
R₁ représente un atome d'hydrogène;
R₂ représente un groupement cyclopropyle;
R₄ représente un atome d'halogène, un groupement nitrile, un groupement nitro, un groupement -CF₃, un groupement -C₂F₅, un groupement -OCF₃ ou un groupement -SCF₃;
et R₅ représente un groupement méthyle.

Les composés particulièrement préférés selon l'invention sont ceux dans lesquels
R₄ représente un atome d'halogène ou un groupement nitrile, un groupement nitro ou un groupement -CF₃; et
R₅ représente un groupement méthyle.

Les composés plus particulièrement préférés selon l'invention sont ceux dans lesquels
R₄ représente un atome de brome ou de chlore ou un groupement nitrile ou -CF₃; et
R₅ représente un groupement méthyle.

Parmi les composés préférés selon l'invention, on peut citer :
le 5-cyclopropyl-N-(4-bromo-3-méthylphényl)-isoxazole-4-carboxamide;
le 5-cyclopropyl-N-(4-chloro-3-méthylphényl)-isoxazole-4-carboxamide;
le 5-cyclopropyl-N-(4-cyano-3-méthylphényl)-isoxazole-4-carboxamide;
le 5-cyclopropyl-N-(4-trifluorométhyl-3-phénylméthyl)-isoxazole-4-carboxamide;
et leurs sels.

L'invention a également pour objet un procédé de préparation des nouveaux phényl carboxamides isoxazoles tels que définis par la formule (I) ci-dessus ainsi que de leurs sels, comprenant la réaction d'un composé de formule (II) 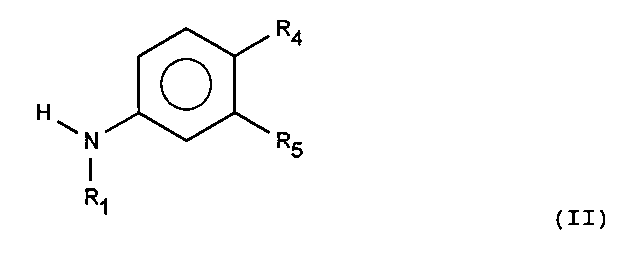 (dans laquelle R₁, R₄ et R₅ sont tels que définis ci-dessus) avec un composé de formule (III) 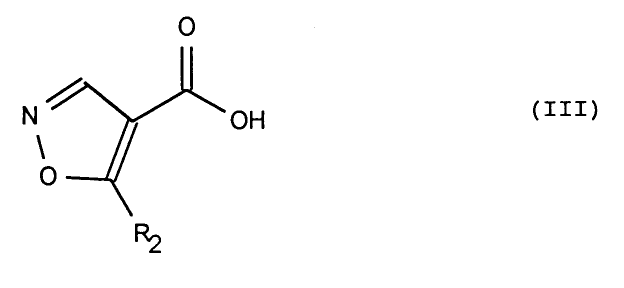 (dans laquelle R₂ est tel que défini ci-dessus) ou un dérivé fonctionnel approprié de celui-ci.

La réaction entre le composé de formule (II) et le composé de formule (III) est de préférence effectuée en présence d'un solvant organique anhydre, tel que le dichlorométhane ou le tétrahydrofurane, et en présence d'un agent de couplage, tel que le dicyclohexylcarbodiimide ou le diisopropylcarbodiimide, à température ambiante.

La réaction entre le composé de formule (II) et un dérivé fonctionnel du composé de formule (III) est de préférence effectuée en présence d'un solvant organique anhydre, tel que le dichlorométhane ou l'éther diéthylique, en présence d'une base organique, telle que la pyridine ou la triéthylamine, et à basse température.

Dans le cas où un dérivé fonctionnel d'un composé de formule (III) est utilisé, il s'agit de préférence d'un halogénure d'acide, notamment d'un chlorure d'acide. Ce chlorure d'acide peut être par exemple préparé in situ, par réaction d'un composé de formule (III) avec du chlorure de thionyle ou du pentachlorure de phosphore.

Les composés de formule (II) utilisés dans le procédé ci-dessus sont généralement des produits connus ou peuvent être préparés par diazotation puis réduction des nitrobenzènes correspondants selon des procédés connus en soi. Les nitroanilines utilisées peuvent être préparées comme indiqué par exemple dans T.P. Sura et al., Synthetic Communications (1988), 18, (16-17) 2161-5. Certaines anilines de formule (II) peuvent être préparées selon les procédés décrits dans le brevet européen EP-A-206951 ou par réduction des nitrobenzènes correspondants.

Les composés de formule (III) utilisés dans le procédé ci-dessus sont également, en général, des produits connus ou peuvent être préparés par des procédés décrits dans la littérature, par exemple dans EP-A-326107.

Les composés selon l'invention présentent des propriétés pharmacologiques intéressantes, et en particulier une remarquable activité anti-proliférative, anti-inflammatoire et anti-tumorale. Ils inhibent d'une part les phénomènes inflammatoires provoqués par des agents irritants et, d'autre part les réactions d'hypersensibilité retardée, en empêchant l'activation des cellules immunitaires par un antigène spécifique.

Ces propriétés sont illustrées plus loin dans la partie expérimentale.

Ces propriétés justifient l'utilisation des nouveaux phényl carboxamide isoxazoles de formule (I) ainsi que de leurs sels pharmaceutiquement acceptables à titre de médicaments.

L'invention a aussi également pour objet l'application à titre de médicaments des nouveaux composés de formule (I) tels que définis ci-dessus et de leurs sels pharmaceutiquement acceptables.

Parmi les médicaments, objet de l'invention, on retient notamment les médicaments caractérisés en ce qu'ils sont constitués par les nouveaux phényl carboxamide isoxazoles de formule (I) ci-dessus dans lesquels R₅ représente un groupement méthyle. Il a été montré que la présence de ce substituant méthyle se traduit par le fait que ces composés présentent une demie-vie métabolique plus courte que les composés correspondants sans substituant méthyle ; ceci est illustré plus amplement dans la partie expérimentale. Par conséquent, de tels composés dans lesquels R₅ représente un groupement méthyle représentent des exemples supplémentaires des composés particulièrement préférés selon l'invention pour une utilisation en tant que médicaments.

Ces médicaments sont utiles par exemple dans le traitement de tumeurs, de la polyarthrite rhumatoïde et des maladies inflammatoires chroniques d'origine immunitaire ou non immunitaire (par ex. la maladie homoloque, les réactions de transplantation, l'uvéite, le psoriasis, le cancer), le diabète, la prévention du rejet aigü et chronique suivant les transplantations d'organes et la resténose post-angioplastie.

La dose habituelle varie selon le composé utilisé, le patient traité et la maladie en question et peut être par exemple de 0,1 mg à 200 mg par jour par voie orale.

L'invention a également pour objet les compositions pharmaceutiques qui renferment à titre de principe actif au moins un composé de formule (I) tel que défini ci-dessus ou un sel pharmaceutiquement acceptable en association avec un ou plusieurs supports et/ou excipients pharmaceutiques.

Pour une utilisation à titre de médicaments, les composés de formule (I) et leurs sels peuvent être incorporés dans des compositions pharmaceutiques destinées à l'administration par voie orale, rectale ou parentérale.

Ces compositions pharmaceutiques peuvent être par exemple solides ou liquides et peuvent être sous des formes utilisées de manière habituelle en médecine humaine telles que: des comprimés simples ou pelliculés, des capsules (dont des gellules), des granulés, des suppositoires, des solutions par ex. injectables; elles peuvent être préparées selon des méthodes classiques. Le(s) principe(s) actif(s) peuvent être incorporés avec des excipients utilisés de manière classique dans les compositions pharmaceutiques tel que le talc, la gomme arabique, l'amidon, le lactose, le stéarate de magnésium, le beurre de cacao, les véhicules aqueux ou non aqueux, les corps gras d'origine animale ou végétale, les dérivés de paraffine, les glycols, divers agents mouillants, dispersants ou émulsionnants et conservateurs.

L'invention a encore pour objet une méthode de traitement des tumeurs, de la polyarthrite rhumatoïde et des maladies inflammatoires chroniques d'origine immunitaire ou non immunitaire chez un sujet humain ou animal comprenant l'administration au sujet d'une quantité efficace d'un composé de formule (I) tel que défini ci-dessus ou d'un sel pharmaceutiquement acceptable.

L'invention est encore illustrée par les exemples non limitatifs suivant:

### Exemple 1: Le 5-cyclopropyl-N-(4-bromo-3-méthylphényl)isoxazole-4-carboxamide

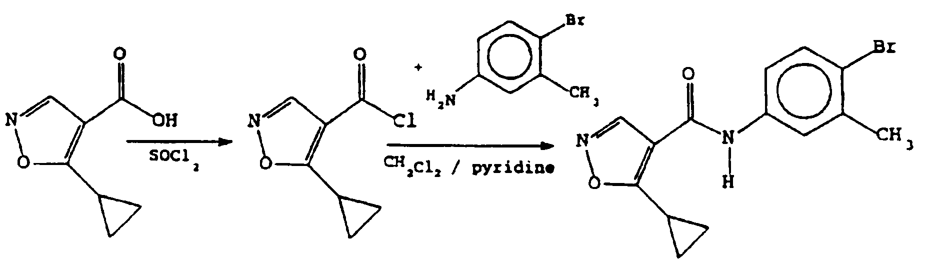

De l'acide 5-cyclopropylisoxazole-4-carboxylique (3,33 g, 21,7 mmoles) et du chlorure de thionyle (25 ml) sont chauffés au reflux pendant 90 minutes. Le chlorure de thionyle est évaporé sous pression réduite et le résidu traité azéotropiquement deux fois par du toluène. De la 4-bromo-3-méthyl-aniline (4,0 g, 21,7 mmoles) est dissoute dans du dichlorométhane sec (125 ml) et refroidie à 0°C. Le chlorure d'acide préparé ci-dessus et de la pyridine (2,4 g, 30 mmoles), dissous chacun dans du dichlorométhane (10 ml), sont ajoutés goutte à goutte simultanément. Après agitation pendant trente minutes à 0°C, le mélange réactionnel est lavé à l'eau salée (3 × 75 ml), séché (MgSO₄) et le dichlorométhane éliminé sous pression réduite pour donner une huile brune. La chromatographie éclair sur colonne (MeOH 2 %, CH₂Cl₂ 98 %) donne une huile brune pâle qui cristallise au repos. Après recristallisation dans de l'éthanol/eau, on obtient le produit en aiguilles crème, F = 133-135°C.

De manière similaire à l'exemple 1, les composés suivants ont été préparés, en utilisant l'aniline substituée appropriée comme produit de départ.

### Exemple 2: Le 5-cylcopropyl-N-(4-nitrophényl)-isoxazole-4-carboxamide

### Exemple 3: Le 5-cyclopropyl-N-(4-cyanophényl)-isoxazole-4-carboxamide

### Exemple 4: Le 5-cyclopropyl-N-(4-chloro-3-méthylphényl)isoxazole-4-carboxamide

### Example 5: Le 5-cyclopropyl-N-(4-cyano-3-méthylphényl)-isoxazole-4-carboxamide

### Exemple 6: Le 5-cyclopropyl-N-(3-méthyl-4-trifluorométhylphényl)-isoxazole-4-carboxamide

Les données spectrales, les rendements, les points de fusion, et les données analytiques pour les exemples 1 à 6 sont données au tableau I.

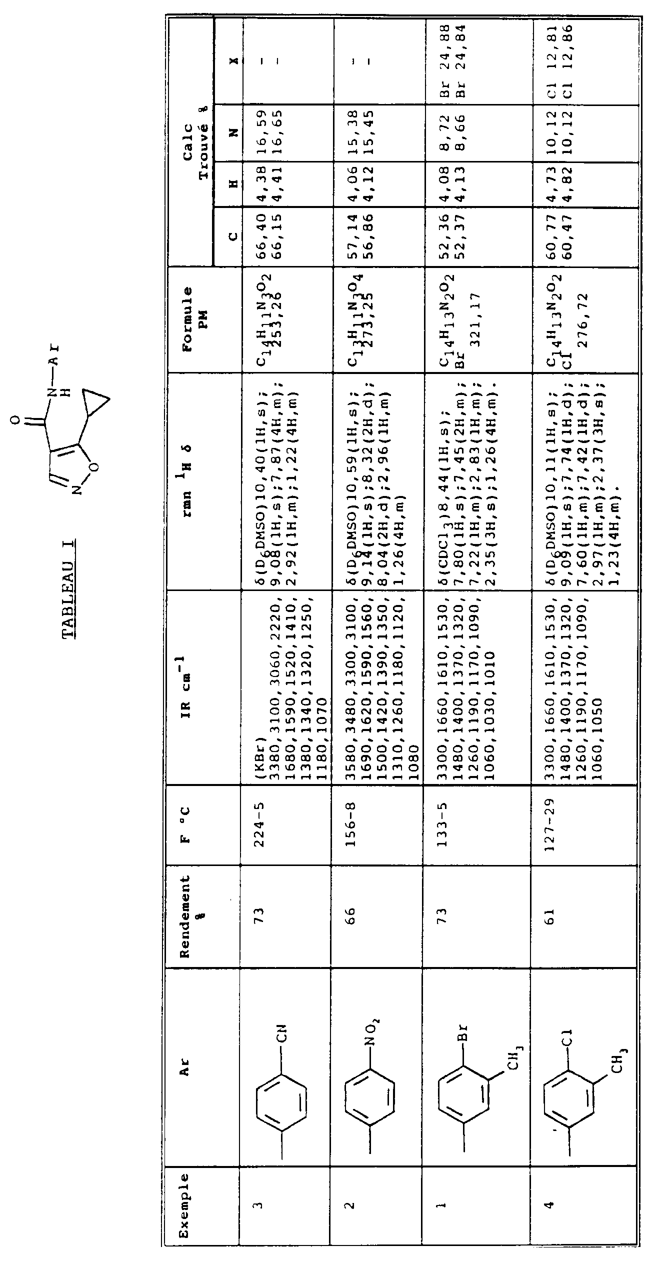

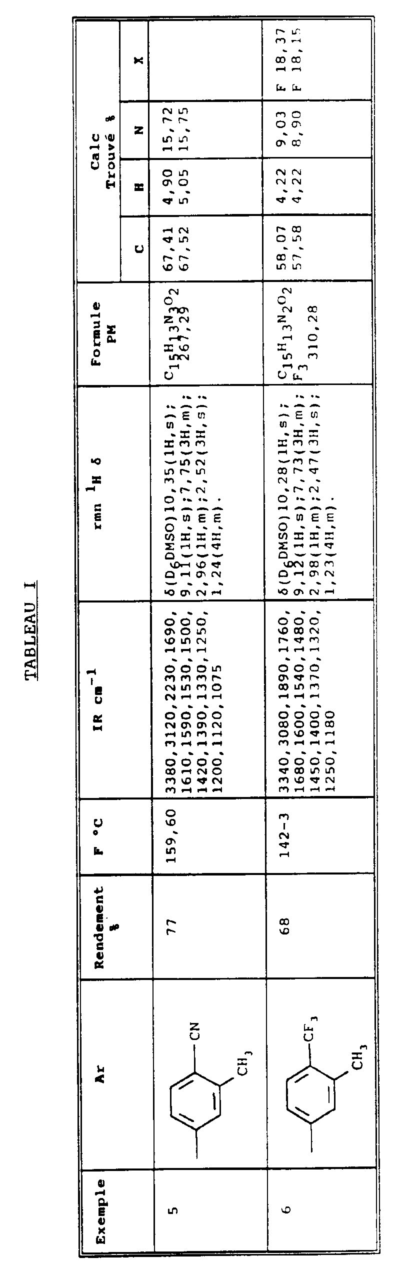

### Exemple 7:

Des comprimés ont été préparés selon la formule suivante:

| | |
|---|---|
| Composé de l'exemple | 120 mg |
| Excipient pour un comprimé jusqu'à | 150 mg |
| (détails concernant l'excipient: lactose, amidon, talc, stéarate de magnésium). | |

### ACTIVITE PHARMACOLOGIQUE

### Méthodes de tests biochimiques

### Oedème des pattes de rat induit par de la carragénine (PO-R) (Test 1)

Une heure après l'administration par voie orale des composés à tester ou du véhicule témoin à des groupes de rats (n=6-12, mâle CFHB, gamme de poids 160-180 g) 1 mg de carragénine dissoute dans 0,2 ml de solution saline est injecté dans le coussinet de la patte arrière droite. Les pattes contralatérales reçoivent des injections témoins de solution saline. Les réponses d'oedème des pattes sont évaluées trois heures plus tard.

### Oedème d'hypersensibilité de type retard des pattes de souris (DTH-M) (Test 2)

Des groupes de souris (n=8-10, male CD-1, gamme de poids 25-30 g) sont sensibilisés par une injection sous-cutanée de 1 mg de sérum albumine bovine méthylée (MBSA) dans des volumes de 0,2 ml d'émulsion de solution saline/émulsion d'adjuvant complet de Freund (FCA). Des groupes de témoins négatifs reçoivent des injections d'émulsion de solution saline/FCA. Les réponses d'oedème DTH des pattes sont évaluées vingt-quatre heures après l'épreuve du coussinet de la patte arrière droite par 0,1 mg de MBSA dans des volumes de 0,05 ml de solution saline au jour sept après la sensibilisation. Les pattes contralatérales reçoivent des injections témoins de solution saline. Les composés à tester ou les véhicules témoins sont administrés par voie orale une fois par jour aux jours quatre, cinq, six et deux fois au jour sept, une heure avant et six heures après l'épreuve par MBSA.

### Oedème d'hypersensibilité de type retard des pattes de rat (DTH-R) (Test 3)

Des groupes de rats (n=8-12, mâle CFHB, gamme de poids 160-180 g) sont sensibilisés par injection sous-cutanée à la base de la queue avec des volumes de 0,1 ml de FCA. Des groupes de témoins négatifs reçoivent une injection d'adjuvant incomplet de Freund. Les réponses d'oedème DTH des pattes sont évaluées vingt-quatre heures après l'épreuve du coussinet de la patte arrière droite par 0,4 mg d'antigène extrait de Mycobacterium tuberculosis dans 0,2 volume de solution saline au jour sept après la sensibilisation. Les pattes contralatérales reçoivent des injections témoins de solution saline.

Les composés à tester sont administrés par voie orale une fois par jour aux jours quatre, cinq, six et deux fois au jour sept, une heure avant et six heures après l'épreuve antigénique.

Les résultats de ces tests sont donnés dans le tableau II.

Les doses sont données en mg/kg p.o.

**Tableau II**

| Exemple | Test 1 | | Test 1 | | Test 3 | |
|---|---|---|---|---|---|---|
| | % inhibition | Dose | % inhibition | Dose | % inhibition | Dose |
| 1 | 12 | 50 | 41 | 100 | 24 | 50 |
| 2 | 0 | 50 | 19 | 100 | 52 | 50 |
| 3 | 43 | 50 | 43 | 30 | 60 | 10 |
| 4 | 11 | 50 | 64 | 100 | 32 | 50 |
| 5 | 18 | 50 | 41 | 30 | 62 | 10 |
| 6 | 12 | 50 | 34 | 100 | 57 | 50 |

### Arthrite adjuvant (Test 4)

Les investigations ont été effectuées par la méthode de Pearson (Arthrit. Rheum. 2, (1959) p. 44). Les animaux expérimentaux utilisés sont des rats mâles d'une souche Wistar-Lewis ayant un poids corporel compris entre 130 et 200 g. Les composés à tester sont administrés par voie orale (p.o.) une fois par jour pendant 12 jours, en commençant au jour de l'immunisation. Les animaux d'un groupe témoin n'ont reçu que le véhicule. Chaque groupe de préparation et de témoins comprend 6 animaux. Le critère utilisé pour déterminer l'activité est le pourcentage de réduction dans l'augmentation du volume des pattes comparativement à celui du groupe témoin non traité.

### Réaction d'Arthus passive inverse (Test 5)

Une heure avant l'épreuve, les composés à tester sont administrés par voie orale à des rats Wistar (mâles ou femelles, 120-200 g). Après cette période de pré-traitement, la réaction est induite par une injection sous-plantaire de sérum de chèvre anti-rat dans la patte arrière droite. La patte arrière gauche, qui sert de témoin, est traitée par une injection de solution saline seule.

Les volumes des pattes sont mesurés immédiatement et quatre heures après l'épreuve par l'anti-corps.

Les résultats des ces tests sont donnés dans le tableau III. Les doses sont données en mg/kg p.o.

**Tableau III**

| Exemple | Test 4 | | Test 5 | |
|---|---|---|---|---|
| | Dose | % inhibition | Dose | % inhibition |
| 1 | 25 | -16 | 50 | 42 |
| | | | 100 | 26 |
| 2 | 25 | 92 | 50 | - 9 |
| | | | 100 | 27 |
| 3 | 25 | 90 | 50 | 41 |
| | | | 100 | 36 |
| 4 | 25 | 22 | 50 | 17 |
| | | | 100 | 28 |
| 5 | 25 | 88 | 50 | 23 |
| | | | 100 | 34 |
| 6 | 25 | 42 | 50 | 24 |
| | | | 100 | 38 |

### DONNEES CINETIQUES

On a fait une comparaison entre un composé connu, à savoir le 5-cyclopropyl-N-(4-trifluorométhylphényl)-isoxazole-4-carboxamide et des composés de la présente invention, à savoir les composés des exemples 5 et 6 ci-dessus.

Les composés ont été administrés par voie orale à des souris à des doses de 30 mg/kg et on a suivi les concentrations plasmatiques pendant 72 heures.

Les résultats montrent que la demi-vie métabolique appropriée pour le composé connu est d'approximativement 21,4 hrs alors que celles pour les deux composés des exemples 5 et 6 de la présente invention était environ un tiers de cette valeur, à savoir 7,1 hrs (ex. 5) et 8,2 hrs (ex. 6).

Ces résultats démontrent l'avantage cinétique conféré par la présence du groupement méthyle représentant le substituant R₅ dans les composés de la présente invention.

## Revendications

1. Composés de formule (I): 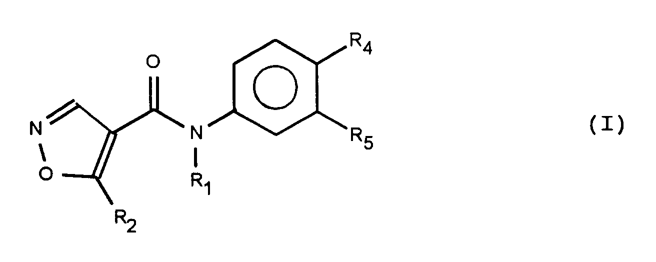 [dans laquelle
R₁ représente un atome d'hydrogène ou un groupement alkyle contenant 1 à 3 atomes de carbone;
R₂ représente un groupement cycloalkyle contenant 3 à 6 atomes de carbone;
R₄ représente un atome d'halogène, un groupement nitrile, un groupement nitro, un groupement -SCH₃, un groupement alkylcarbonyle en C₁₋₆, un groupement cycloalkylcarbonyle en C₃₋₆, un groupement -CX₃, un groupement -WCX₃, un groupement -(CH₂)ₙCX₃, un groupement -(CX₂)ₙCX₃, un groupement -W(CX₂)ₙCX₃ ou un groupement -W(CH₂)ₙCX₃,
dans lesquels X représente un atome d'halogène, W représente un atome d'oxygène ou de soufre, et n représente 1, 2 ou 3; et
R₅ représente un groupement méthyle ;
et leurs sels.

2. Composés selon la revendication 1, dans lesquels
R₁ représente un atome d'hydrogène;
R₂ représente un groupement cyclopropyle;
R₄ représente un atome d'halogène, un groupement nitrile, un groupement nitro, un groupement -CF₃, un groupement -C₂F₅, un groupement -OCF₃ ou un groupement -SCF₃;
et R₅ représente un groupement méthyle.

3. Composés selon la revendication 1 ou la revendication 2, dans lesquels
R₄ représente un atome d'halogène ou un groupement nitrile, un groupement nitro ou un groupement -CF₃; et
R₅ représente un groupement méthyle.

4. Composés selon l'une quelconque des revendications 1 à 3, dans lesquels
R₄ représente un atome de brome ou de chlore ou un groupement nitrile ou -CF₃; et
R₅ représente un groupement méthyle.

5. Composés selon l'une quelconque des revendications 1 à 5, sélectionnés parmi:
le 5-cyclopropyl-N-(4-bromo-3-méthylphényl)-isoxazole-4-carboxamide;
le 5-cyclopropyl-N-(4-chloro-3-méthylphényl)-isoxazole-4-carboxamide;
le 5-cyclopropyl-N-(4-cyano-3-méthylphényl)-isoxazole-4-carboxamide;
le 5-cyclopropyl-N-(4-trifluorométhyl-3-méthylphényl)-isoxazole-4-carboxamide;
et leurs sels.

6. Procédé de préparation d'un composé de formule (I) selon l'une quelconque des revendications 1 à 5, comprenant la réaction d'un composé de formule (II) 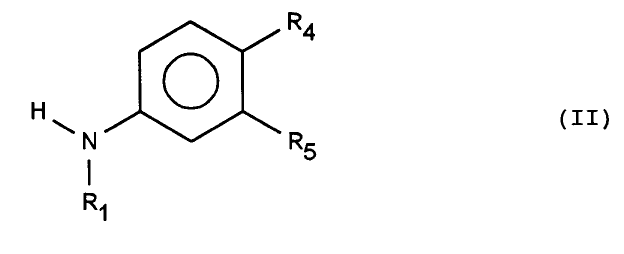 (dans laquelle R₁, R₄ et R₅ sont tels que définis dans la revendication 1 avec un composé de formule (III) 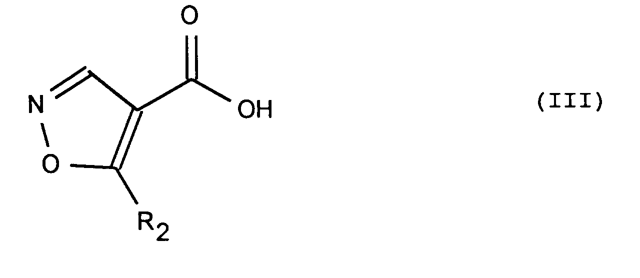 (dans laquelle R₂ est tel que défini dans la revendication 1) ou un dérivé fonctionnel convenable de celui-ci.

7. Médicament constitué par les nouveaux composés de formule (I) selon l'une quelconque des revendications 1 à 5 ainsi que par leurs sels pharmaceutiquement acceptables.

8. Médicament constitué par les nouveaux composés de formule (I) selon l'une quelconque des revendications 1 à 4, dans lequel R₅ représente un groupement méthyle ainsi que par leurs sels pharmaceutiquement acceptables.

9. Compositions pharmaceutiques comprenant comme principe actif au moins un composé de formule (I) selon l'une quelconque des revendications 1 à 5 ou un sel pharmaceutiquement acceptable en association avec un ou plusieurs supports et/ou excipients pharmaceutiques.

## Claims

1. Compounds of formula (I): 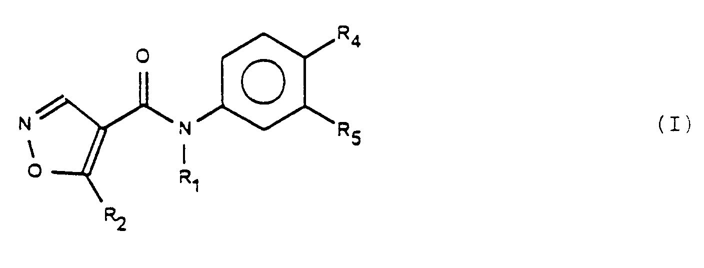 [in which
R₁ represents a hydrogen atom or an alkyl group containing 1 to 3 carbon atoms;
R₂ represents a cycloalkyl group containing 3 to 6 carbon atoms;
R₄ represents a halogen atom, a nitrile group, a nitro group, an -SCH₃ group, a C₁₋₆ alkylcarbonyl group, a C₃₋₆ cycloalkylcarbonyl group, a -CX₃ group, a -WCX₃ group, a -(CH₂)ₙCX₃ group, a -(CX₂)ₙCX₃ group, a -W(CX₂)ₙCX₃ group or a -W(CH₂)ₙCX₃ group,
in which X represents a halogen atom, W represents an oxygen or sulphur atom, and n represents 1, 2 or 3; and
R₅ represents a methyl group;
and their salts.

2. Compounds according to claim 1, in which
R₁ represents a hydrogen atom;
R₂ represents a cyclopropyl group;
R₄ represents a halogen atom, a nitrile group, a nitro group, a -CF₃ group, a -C₂F₅ group, an -OCF₃ group or an -SCF₃ group;
and R₅ represents a methyl group.

3. Compounds according to claim 1 or claim 2, in which
R₄ represents a halogen atom or a nitrile group, a nitro group or a -CF₃ group; and
R₅ represents a methyl group.

4. Compounds according to any one of claims 1 to 3, in which
R₄ represents a bromine or chlorine atom or a nitrile or -CF₃ group; and
R₅ represents a methyl group.

5. Compounds according to any one of claims 1 to 5, selected from:
5-cyclopropyl-N-(4-bromo-3-methylphenyl)-isoxazole-4-carboxamide;
5-cyclopropyl-N-(4-chloro-3-methylphenyl)-isoxazole-4-carboxamide;
5-cyclopropyl-N-(4-cyano-3-methylphenyl)-isoxazole-4-carboxamide;
5-cyclopropyl-N-(4-trifluoromethyl-3-methylphenyl)-isoxazole-4-carboxamide;
and their salts.

6. Process for the preparation of a compound of formula (I) according to any one of claims 1 to 5, comprising the reaction of a compound of formula (II) 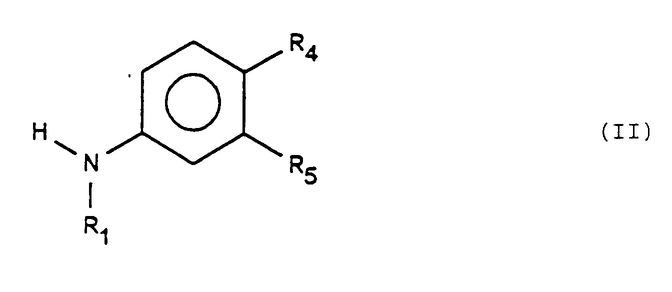 (in which R₁, R₄ and R₅ are as defined in claim 1) with a compound of formula (III) 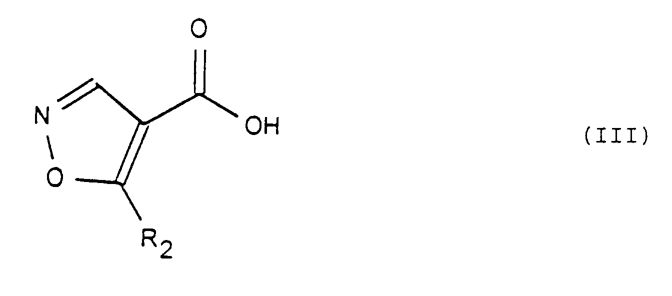 (in which R₂ is as defined in claim 1) or a suitable functional derivative of the latter.

7. Medicament constituted by novel compounds of formula (I) according to any one of claims 1 to 5 as well as their pharmaceutically acceptable salts.

8. Medicament constituted by novel compounds of formula (I) according to any one of claims 1 to 4, in which R₅ represents a methyl group, as well as their pharmaceutically acceptable salts.

9. Pharmaceutical compositions containing as active ingredient at least one compound of formula (I) according to any one of claims 1 to 5 or a pharmaceutically acceptable salt in combination with one or more supports and/or pharmaceutical excipients.

## Patentansprüche

1. Verbindungen der Formel (I) 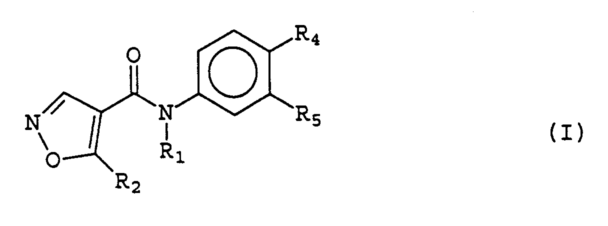 in der
R₁ ein Wasserstoffatom oder eine Gruppe Alkyl mit 1 bis 3 Kohlenstoffatomen darstellt;
R₂ eine Gruppe Cycloalkyl mit 3 bis 6 Kohlenstoffatomen bedeutet;
R₄ ein Halogenatom, eine Gruppe Nitril, eine Gruppe Nitro, eine Gruppe -SCH₃, eine Gruppe Alkylcarbonyl mit 1 bis 6 Kohlenstoffatomen, eine Gruppe Cycloalkylcarbonyl mit 3 bis 6 Kohlenstoffatomen, eine Gruppe -CX₃, eine Gruppe -WCX₃, eine Gruppe -(CH₂)ₙCX₃, eine Gruppe -(CX₂)ₙCX₃, eine Gruppe -W(CX₂)ₙCX₃ oder eine Gruppe -W(CH₂)ₙCX₃ darstellt, worin X ein Halogenatom ist, W ein Sauerstoffatom oder Schwefelatom bedeutet und n 1, 2 oder 3 darstellt, und
R₅ eine Gruppe Methyl ist;
und ihre Salze.

2. Verbindungen nach Anspruch 1, in denen
R₁ ein Wasserstoffatom ist;
R₂ eine Gruppe Cyclopropyl darstellt;
R₄ ein Halogenatom, eine Gruppe Nitril, eine Gruppe Nitro, eine Gruppe -CF₃, eine Gruppe -C₂F₅, eine Gruppe -OCF₃ oder eine Gruppe -SCF₃ bedeutet; und
R₅ eine Gruppe Methyl ist.

3. Verbindungen nach Anspruch 1 oder Anspruch 2, in denen
R₄ ein Halogenatom oder eine Gruppe Nitril, eine Gruppe Nitro oder eine Gruppe -CF₃ darstellt; und
R₅ eine Gruppe Methyl ist.

4. Verbindungen nach irgendeinem der Ansprüche 1 bis 3, in denen
R₄ ein Bromatom oder Chloratom oder eine Gruppe Nitril oder eine Gruppe -CF₃ darstellt; und
R₅ eine Gruppe Methyl ist.

5. Verbindungen nach irgendeinem der Ansprüche 1 bis 4, ausgewählt unter:
5-Cyclopropyl-N-(4-brom-3-methylphenyl)-isoxazol-4-carboxamid;
5-Cyclopropyl-N-(4-chlor-3-methylphenyl)-isoxazol-4-carboxamid;
5-Cyclopropyl-N-(4-cyano-3-methylphenyl)-isoxazol-4-carboxamid;
5-Cyclopropyl-N-(4-trifluormethyl-3-methylphenyl)-isoxazol-4-carboxamid;
und ihren Salzen.

6. Verfahren zur Herstellung einer Verbindung der Formel (I) nach irgendeinem der Ansprüche 1 bis 5, umfassend die Reaktion einer Verbindung der Formel (II) 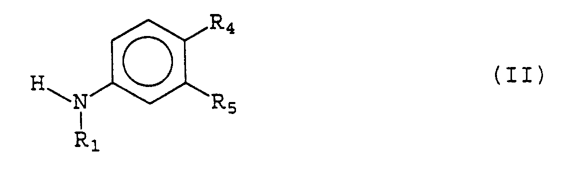 in der R₁, R₄ und R₅ wie in Anspruch 1 definiert sind, mit einer Verbindung der Formel (III) 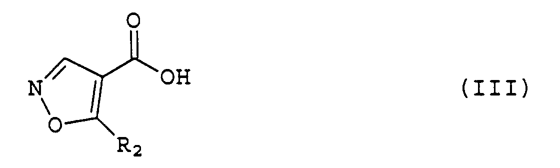 oder einem geeigneten funktionellen Derivat von diesem, worin R₂ wie in Anspruch 1 definiert ist.

7. Arzneimittel, bestehend aus den neuen Verbindungen der Formel (I) nach irgendeinem der Ansprüche 1 bis 5 sowie aus ihren pharmazeutisch akzeptablen Salzen.

8. Arzneimittel, bestehend aus den neuen Verbindungen der Formel (I) nach irgendeinem der Ansprüche 1 bis 4, worin R₅ eine Gruppe Methyl darstellt, sowie aus ihren pharmazeutisch akzeptablen Salzen.

9. Pharmazeutische Zusammensetzungen, umfassend als Wirkstoff mindestens eine Verbindung der Formel (I) nach irgendeinem der Ansprüche 1 bis 5 oder ein pharmazeutisch akzeptables Salz in Assoziation mit einem oder mehreren pharmazeutischen Trägern und/oder Füllstoffen.
